# EUROPÄISCHE PATENTANMELDUNG

(11) **EP 2 765 184 A1**
(43) Veröffentlichungstag der Anmeldung: **13.08.2014**
(21) Anmeldenummer: 13000723.0
(22) Anmeldetag: 12.02.2013
(51) Int. Cl.: C12M 1/00, F26B 23/00

(54) **Gärrest-Konditionierer und Verfahren zur Konditionierung von Gärresten**

(71) Anmelder: KOMPOFERM GmbH, 33428 Marienfeld (DE)
(72) Erfinder: Eggersmann, Karlgünter, 33428 Marienfeld (DE)
(74) Vertreter: Schober, Mirko

(57) **Zusammenfassung**

Ein Gärrest-Konditionierer (1), der zur Konditionierung von Gärresten (3) oder Klärschlamm (3) oder organischen Restmassen mit hohem Wassergehalt dient, umfasst eine sich in einer ersten Erstreckungsrichtung (X) erstreckende Ablagefläche (2) für Gärreste (3). Er weist ferner eine Gärrest-Aufgabestelle (4) und eine von der Gärrest-Aufgabestelle (4) entlang der ersten Erstreckungsrichtung (X) beabstandete Gärrest-Entnahmestelle (5) sowie eine Umschicht- und Auflockerungseinrichtung (6) zur Umschichtung, Aulockerung, zum Aufschluss oder/und Homogenisieren auf der Ablagefläche (2) befindlicher Gärreste (3) auf. Die Umschicht- und Auflockerungseinrichtung (6) ist geeignet und bestimmt, die auf der Ablagefläche (2) befindlichen Gärreste (3) sowohl umzuschichten als auch entlang der ersten Erstreckungsrichtung (X) zu fördern.

## Beschreibung

Die Erfindung betrifft einen Gärrest-Konditionierer zur Konditionierung von Gärresten oder Klärschlamm sowie ein Verfahren zur Konditionierung von Gärresten oder Klärschlamm.

Gärreste und Klärschlamm fallen beispielsweise in Anlagen zur Biogaserzeugung oder Kläranlagen an und weisen eine für ihre weitere Behandlung, beispielsweise durch Kompostierung, ungünstige Konsistenz, insbesondere einen zu hohen Feuchtigkeitsgehalt, auf.

Der Erfindung liegt daher die Aufgabe zugrunde, einen Gärrest-Konditionierer zur Aufbereitung von Gärresten oder Klärschlamm sowie ein Verfahren zur Konditionierung von Gärresten oder Klärschlamm oder organischen Restmassen mit hohem Wassergehalt aufzuzeigen, welche eine effiziente Konditionierung der Gärreste unter kontrollierter Freisetzung von Emissionen ermöglichen.

Die Aufgabe wird gelöst durch einen Gärrest-Konditionierer und ein Verfahren zur Konditionierung von Gärresten oder Klärschlamm nach den unabhängigen Ansprüchen 1 und 10. Die abhängigen Ansprüche betreffen vorteilhafte Ausführungsformen.

Im Folgenden wird für die Begriffe "Gärreste" und "Klärschlamm" sowie für "organische Restmassen mit hohem Wassergehalt" lediglich der Begriff "Gärreste" verwendet. Dies gilt auch für die Ansprüche und dient der sprachlichen Vereinfachung.

Der erfindungsgemäße Gärrest-Konditionierer umfasst eine sich in einer ersten Erstreckungsrichtung erstreckende Ablagefläche für Gärreste. Auf dieser können die Gärreste zu einem Haufwerk aufgeschichtet werden. Weiterhin umfasst er eine Gärrest-Aufgabestelle und eine von der Gärrest-Aufgabestelle entlang der ersten Erstreckungsrichtung beabstandete Gärrest-Entnahmestelle. Gärrest-Aufgabestelle und Gärrest-Entnahmestelle bezeichnen dabei zunächst nur die räumlich festgelegten Bereiche, in denen die Gärreste auf den Gärrest-Konditionierer aufgegeben bzw. von diesem entnommen werden. Eine spezielle Ausgestaltung dieser kann vorteilhaft sein, ist jedoch grundsätzlich nicht notwendig. Gärrest-Aufgabestelle und Gärrest-Entnahmestelle müssen sich folglich konstruktiv nicht zwangsläufig vom Rest des Gärrest-Konditionierers, insbesondere anderen Abschnitten des Gärrest-Konditionierers entlang der ersten Erstreckungsrichtung unterscheiden.

Der erfindungsgemäße Gärrest-Konditionierer ist zur Optimierung des thermischen Wirkungsgrades infolge von Wärmeverlusten an die Atmosphäre und Rückkondensation bevorzugt wärmegedämmt ausgebildet.

Weiterhin weist der erfindungsgemäße Gärrest-Konditionierer eine Umschicht- und Auflockerungseinrichtung auf. Diese Umschicht- und Auflockerungseinrichtung ist geeignet und bestimmt, die bei bestimmungsgemäßer Verwendung auf der Ablagefläche befindlichen Gärreste sowohl umzuschichten als auch entlang der ersten Erstreckungsrichtung zu fördern und aufzulockern.

Durch diese Doppelfunktionalität der Umschicht- und Auflockerungseinrichtung wird die Konstruktion eines kostengünstigen Gärrest-Konditionierers ermöglicht.

Das erfindungsgemäße Verfahren sieht vor, dass die Gärreste im Bereich der Gärrest-Aufgabestelle einem Gärrest-Konditionierer aufgegeben werden und im Bereich einer von der Gärrest-Aufgabestelle entlang einer ersten Erstreckungsrichtung einer Ablagefläche für die Gärreste des Gärrest-Konditionierers beabstandeten Gärrest-Entnahmestelle dem Gärrest-Konditionierer entnommen werden, wobei die Gärreste von der Gärrest-Aufgabestelle zur Gärrest-Entnahmestelle entlang der ersten Erstreckungsrichtung über die Ablagefläche gefördert werden, wobei eine Umschicht- und Auflockerungseinrichtung sowohl die Förderung als auch eine Umschichtung, Auflockerung und/oder Aufschluss der Gärreste bewirkt. Das erfindungsgemäße Verfahren sieht folglich vor, mittels einer Umschicht- und Auflockerungseinrichtung sowohl die Umschichtung als auch die Förderung der Gärreste zu bewirken, was den Vorteil hat, dass keine separaten Einrichtungen zur Erfüllung der beiden Funktionen benötigt werden. Dabei ist es vorteilhaft, wenn die Umschicht- und Auflockerungseinrichtung zeitgleich nur auf einen Teilabschnitt der Gesamterstreckung der Ablagefläche entlang der ersten Erstreckungsrichtung zwischen Gärrest-Aufgabestelle und Gärrest-Entnahmestelle auf die Gärreste einwirkt. Die Umschicht- und Auflockerungseinrichtung, die sich dabei nur über einen Teilabschnitt der genannten Gesamterstreckung erstreckt, kann auf diese Weise konstruktiv kleiner, und damit Material und Kosten sparender ausgeführt werden, als eine Umschicht- und Auflockerungseinrichtung, welche auf die gesamte Gärrestmenge gleichzeitig einwirken könnte, sich dazu also über die gesamte Ablagefläche erstrecken müsste.

Dieser vorteilhaften Maßnahme liegt die Erkenntnis zugrunde, dass es im Hinblick auf den Konditionierungsprozess ausreichend ist, wenn nur jeweils ein Teil der Gärreste zeitgleich umgeschichtet wird, da insbesondere die Trocknungsvorgänge eine gewisse Zeitdauer beanspruchen. Das mehrfache Umschichten, welches dazu dient, die Gärrestmasse zu homogenisieren und insbesondere Verklumpungen aufzubrechen, beansprucht nur einen Bruchteil der Zeit, die der gesamte Konditionierungsprozess andauert. Ebenso wird nur eine niedrige Fördergeschwindigkeit benötigt, so dass es ausreichend ist, zeitgleich jeweils nur einen Teil der auf der Ablagefläche aufgehäuften Gärreste gleichzeitig in Richtung von der Gärrest-Aufgabestelle weg zur Gärrest-Entnahmestelle hin zu bewegen.

Dies wird insbesondere durch eine Umschicht- und Auflockerungseinrichtung ermöglicht, die einen rotierenden Umschichtkörper aufweist. Die Umschicht- und Auflockerungseinrichtung wirkt über diesen Umschichtkörper, der vorzugsweise um eine horizontale und/oder rechtwinklig zur ersten Erstreckungsrichtung verlaufende Achse rotiert, auf die Gärreste ein. Dabei sorgt der Umschichtkörper einerseits für eine Umschichtung als auch für einen Transport, wobei es besonders vorteilhaft ist, wenn die Rotationsrichtung des Umschichtkörpers so gewählt ist, dass sich dessen oberer Scheitelpunkt in Richtung der Gärrest-Entnahmestelle bewegt. Weiter wird durch diese Umschichtung beim mehrfachen Umschichten ein Gärrestklumpen nach und nach aufgebrochen, derart, dass äußeres bereits angetrocknetes Material abfällt und darunter befindliches noch feuchtes Material weiter angetrocknet wird, wobei sich dieser Vorgang wiederholt. Zudem werden Klumpen geteilt, so dass sich größere Oberflächen zur Trocknung einstellen. Unter einem Scheitelpunkt ist auch eine sich insbesondere bei einem walzenförmig ausgestalteten Gärrestkörper entstehende Scheitellinie zu verstehen.

Um es zu ermöglichen, dass die Umschicht- und Auflockerungseinrichtung zwar nicht zeitgleich aber zumindest zeitversetzt auf alle Gärreste auf der Ablage einwirken kann, ist es vorteilhaft, wenn die Umschicht- und Auflockerungseinrichtung entlang der ersten Erstreckungsrichtung verfahrbar ist.

Dies kann beispielsweise durch sich entlang der ersten Erstreckungsrichtung erstreckende Führungen, beispielsweise durch ein Schienensystem erreicht werden. Dabei befinden sich die Führungen vorteilhafterweise oberhalb der bei bestimmungsgemäßer Verwendung des Gärrest-Konditionierers sich ergebenden Haufwerkshöhe der Gärreste. Hierdurch wird sichergestellt, dass die Funktion der Führungen für die Umschicht- und Auflockerungseinrichtung nicht durch Gärreste beeinträchtigt wird.

Es ist vorteilhaft, das Verfahren so durchzuführen, dass sich eine Haufwerkshöhe der Gärreste von 10 bis 100 cm, insbesondere von 20 bis 40 cm ergibt. Bei dieser Haufwerkshöhe ergibt sich eine Trockungsgeschwindigkeit, bei der sich die Vorteile der vorliegenden Erfindung effizient nutzen lassen.

Es ist sinnvoll, die Umschicht- und Auflockerungseinrichtung am Gärrest-Konditionierer, vorzugsweise an den Führungen, durch eine Abstandsänderungseinrichtung aufzunehmen. Die Abstandsänderungseinrichtung dient zur Veränderung des Abstands der Umschicht- und Auflockerungseinrichtung zur Ablagefläche und kann beispielsweise durch ein Schwenkelement realisiert sein. Hierdurch kann die Umschicht- und Auflockerungseinrichtung harten Gegenständen, die sich ggf. unter den Gärresten befinden, nach oben ausweichen, wodurch Beschädigungen der Umschicht- und Auflockerungseinrichtung bzw. der Ablagefläche durch die harten Gegenstände wirkungsvoll verhindert werden, wenn diese zwischen die Umschicht- und Auflockerungseinrichtung und die Ablagefläche geraten, und die Rückfahrt ermöglicht wird.

Es ist weiterhin vorteilhaft, wenn die Ablagefläche eine Heizeinrichtung aufweist, welche einen direkten Wärmeübertrag an die Gärreste durch Wärmeleitung ermöglicht. Durch die Heizeinrichtung ist es möglich, die Gärreste zu beheizen, was deren Trocknung beschleunigt. Dabei ist insbesondere das Zusammenwirken einer beheizten Ablagefläche mit einer Umschicht- und Auflockerungseinrichtung vorteilhaft, da auf diese Weise mit jeder Umschichtung eine neue Oberfläche der Gärreste in Kontakt mit der beheizten Ablagefläche kommt, so dass sich insgesamt eine homogene und beschleunigte Trocknung ergibt.

Weiterhin vorteilhaft ist es, wenn die Ablagefläche eine Belüftungseinrichtung zur Belüftung der Gärreste aufweist. Durch das Durchlüften der Gärreste wird ein schnellerer Feuchtigkeitsabtransport und auch Wärmeeintrag in die Gärreste gewährleistet. Hierbei ist es insbesondere vorteilhaft, dass durch die Homogenisierung Verklumpungen, sogenannte Mikrochargen, aufgebrochen werden, so dass die Durchlüftung auch die innerhalb dieser Verklumpungen gespeicherte Feuchtigkeit erfassen kann und die Verklumpungen nicht umströmt.

Bevorzugt ist vorgesehen, eine Anlage aus einer Mehrzahl Gärrest-Konditionierern vorzusehen. Die Mehrzahl Gärrest-Konditionierer kann dabei parallel oder/und in Reihe geschaltet sein. Bevorzugt ist eine Variante, bei der aus Bauraumgründen eine Mehrzahl von Gärrest-Kondtionieren räumlich übereinander angeordnet sind.

Der beschriebene Gärrest-Konditionierer bietet nicht nur die Möglichkeit, Gärreste thermisch zu trocknen, sondern stellt insbesondere eine Vorstufe für einen optimierten Start einer nachfolgenden aeroben Behandlung der Gärreste dar.

Mittels des beschriebenen Gärrest-Konditionierers wird in den Gärresten vorhandenes Ammoniak, das für einen aeroben Prozess toxisch ist, mittels vorgeheizter Luft effektiv ausgetrieben und sicher gefasst. Die aufkonzentrierten Abluftströme können einer entsprechenden Abluftbehandlung, z.B. einem sauren Wäscher, zugeführt werden.

Die Behandlung der Gärreste mittels des beschriebenen Gärrest-Konditionierers setzt durch den Luftstrom in den Gärresten vorhandenes Methan frei. Ebenso wird durch die mechanischen Komponenten des beschriebenen Gärrest-Konditionierers (Auflockern, Aufschließen und/oder Homogenisieren) eine Bildung von Methan innerhalb von Gärrestklumpen o.ä. deutlich reduziert bzw. vermieden und die Freisetzung des klimarelevanten Methan an die Atmosphäre in nachgelagerten Prozessen deutlich reduziert.

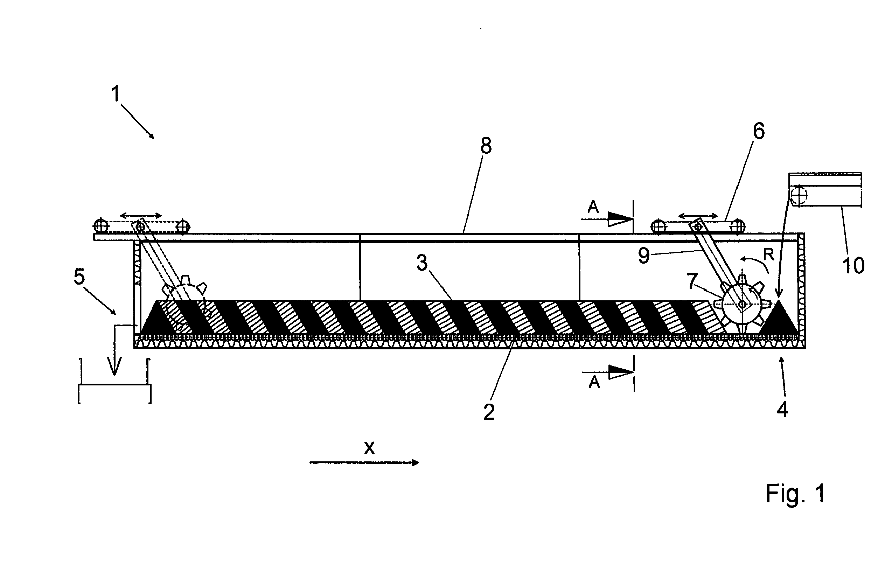

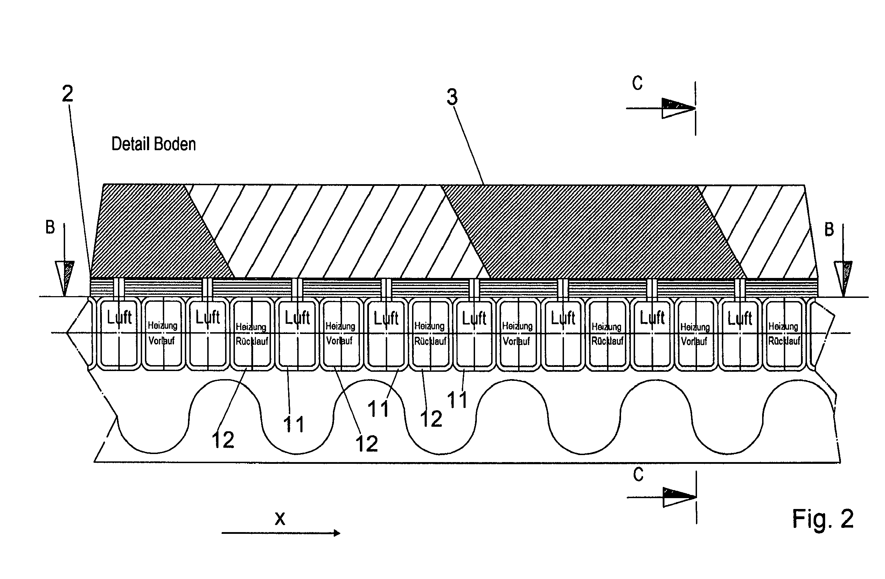

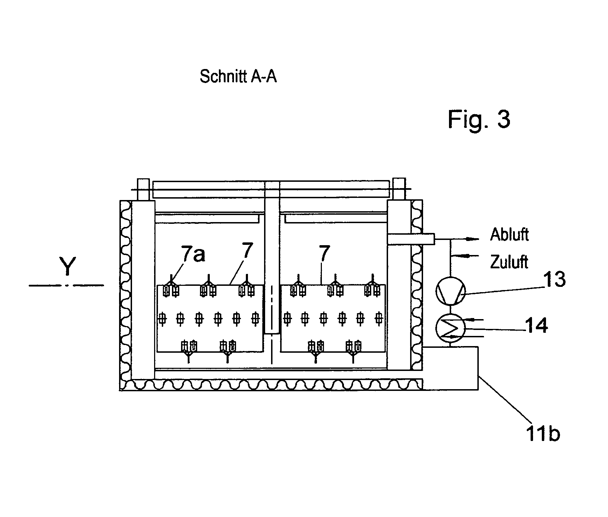

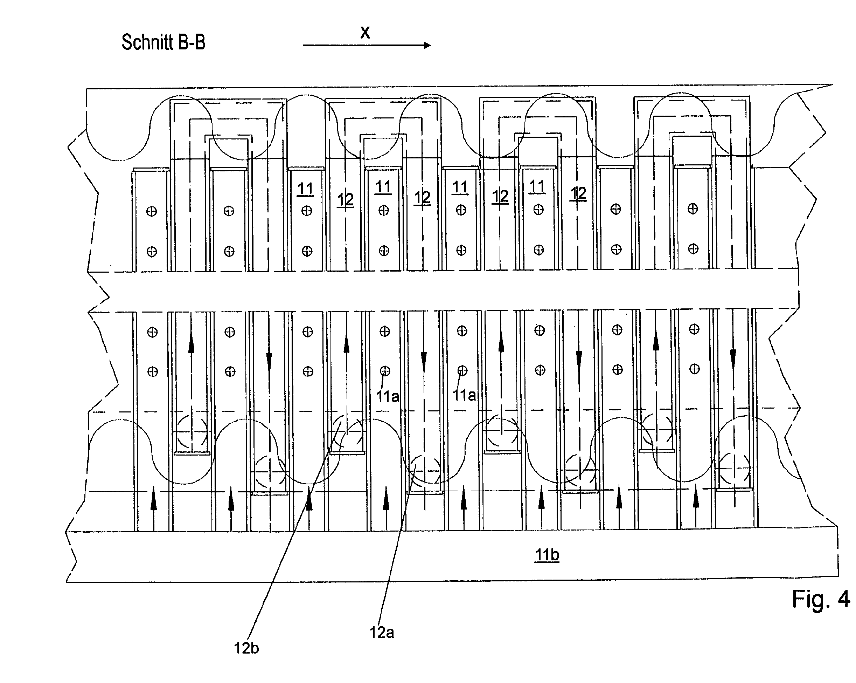

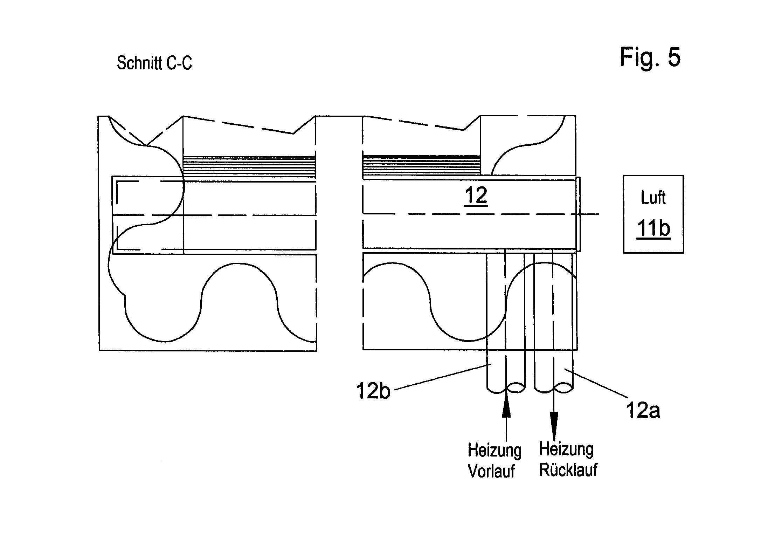

Durch eine optimierte Systematik/Steuerung der Umschicht- und Auflockerungseinheit besteht die Möglichkeit, Volumen- bzw. Schütthöhenverluste der Gärreste in Folge von z.B. oTS-Abbau und/oder erhöhter Feinkornbildung durch die Bearbeitung auszugleichen. Über den Ausgleich der Schütthöhe der Gärreste hinaus kann während der Behandlung die Schütthöhe und damit die Verweilzeit der Gärreste innerhalb des Gärrest-Konditionierers erhöht werden. Dieses führt zu einer Reduktion der Investions- und damit Behandlungskosten. Die Erfindung wird im Folgenden anhand der Figuren 1 bis 5 schematisch näher erläutert.
- Figur 1: - zeigt einen beispielhaften erfindungsgemäßen Gärrest-Konditionierer in einer schematischen Darstellung,
- Figur 2: - zeigt eine Detaildarstellung des Bodens des beispielhaften erfindungsgemäßen Gärrest-Konditionierers in einer Schnittdarstellung,
- Figur 3: - zeigt eine Schnittdarstellung mit einer Schnittrichtung rechtwinklig zur Haupterstreckungsrichtung X durch den beispielhaften erfindungsgemäßen Gärrest-Konditionierer,
- Figur 4: - zeigt eine Schnittdarstellung durch den Boden des Gärrest-Konditionierers in einer horizontalen Schnittebene,
- Figur 5: - zeigt einen Detailschnitt durch den Boden des beispielhaften erfindungsgemäßen Gärrest-Konditionierers.

Der beispielhafte erfindungsgemäße Gärrest-Konditionierer 1 weist eine Ablagefläche 2 für die Gärreste 3 auf. Die Gärreste 3 bilden ein Haufwerk auf der Ablagefläche 2 und werden an der Gärrest-Aufgabestelle 4, z.B. durch einen Förderer, auf die Ablagefläche 2 aufgegeben.

Die Umschicht- und Auflockerungseinrichtung 6, die entlang einem Schienensystem 8, welches oberhalb der Haufwerkshöhe der Gärreste angeordnet ist, aufgenommen ist, kann über einen in der Rotationsrichtung R rotierenden Umschichtkörper 7 auf die Gärreste einwirken, diese dabei umschichten, auflockern und aufbrechen und in Richtung der Gärrest-Entnahmestelle 5 fördern, an der die Gärreste 3 aus dem Gärrest-Konditionierer 1 durch die Umschicht- und Auflockerungseinrichtung 6 ausgetragen werden.

Der Umschichtkörper 7 ist als um die Achse Y rotierende Walze ausgebildet, die Vorsprünge 7a aufweist, durch welche sie eine Profilierung erhält, mit der sie gut in die Gärreste 3 eingreifen und diese homogenisieren, aufbrechen und auflockern und somit schneller trocknen kann.

Der Boden des Gärrest-Konditionierers 1 weist unterhalb der Ablagefläche 2 sowohl eine Heizeinrichtung 12 als auch eine Belüftungseinrichtung 11 auf. Die Heizeinrichtung 12 weist U-förmige, von einem Heizmedium durchströmbare Kanäle auf, die unterhalb der Ablagefläche 2 horizontal verlaufen. Dabei sind die U-förmigen Kanäle jeweils mit einer Vorlaufleitung 12b und einer Rücklaufleitung 12a für das Heizmedium verbunden, die über eine gemeinsame Vorlaufleitung mit Heizmedium versorgt werden, wobei das Heizmedium ebenfalls über eine gemeinsame Rücklaufleitung wieder abgeführt werden kann. Natürlich können auch mehrere Heizkreisläufe mit entsprechenden Mehrzahlen von Vorlaufleitungen oder Rücklaufleitungen vorgesehen sein.

Im gezeigten Beispiel wechseln sich die Kanäle der Heizeinrichtung 12 in Erstreckungsrichtung X mit Kanälen der Belüftungseinrichtung 11 ab. Diese sind mit einer Hauptzuluftleitung 11b verbunden, von der aus die zur Durchlüftung der Gärreste 3 vorgesehene Luft in die sich kammartig von dieser weg erstreckenden Kanäle der Heizeinrichtung 11 gefördert wird, um durch Durchtrittsöffnungen 11a in der Ablagefläche 2 in die Gärreste 3 eingeleitet bzw. durch dieselben durchgeleitet zu werden.

Im gezeigten Beispiel wird die Luft durch eine Fördereinrichtung 13, beispielsweise einen Verdichter, gefördert. Weiterhin ist eine zusätzliche Heizeinrichtung 14 für die zuzuführende Luft vorgesehen, was den Vorteil aufweist, dass so über die Luft zusätzliche Wärme in das Haufwerk der Gärreste 3 eingebracht werden kann. Weiterhin kann die warme Luft eine größere Menge Feuchtigkeit aufnehmen und aus den Gärresten abführen. Insbesondere Gärrest und Klärschlamm wird über die geschilderten Maßnahmen darin enthaltenes Ammoniak sicher ausgetrieben. Zudem werden durch das Aufbrechen der Gärrestklumpen dann noch eventuell ablaufende Methan produzierende Vergärungsprozesse bzw. anaerobe Prozesse sicher abgebrochen.

## Patentansprüche

1. Gärrest-Konditionierer (1), zur Konditionierung von Gärresten (3) oder Klärschlamm (3) oder organischen Restmassen mit hohem Wassergehalt, umfassend eine sich in einer ersten Erstreckungsrichtung (X) erstreckende Ablagefläche (2) für Gärreste (3), eine Gärrest-Auf-. gabestelle (4) und eine von der Gärrest-Aufgabestelle (4) entlang der ersten Erstreckungsrichtung (X) beabstandete Gärrest-Entnahmestelle (5) sowie eine Umschicht- und Auflockerungseinrichtung (6) zur Umschichtung, Aulockerung, zum Aufschluss oder/und Homogenisieren auf der Ablagefläche (2) befindlicher Gärreste (3), wobei die Umschicht- und Auflockerungseinrichtung (6) geeignet und bestimmt ist, die auf der Ablagefläche (2) befindlichen Gärreste (3) sowohl umzuschichten als auch entlang der ersten Erstreckungsrichtung (X) zu fördern.

2. Gärrest-Konditionierer (1) nach Anspruch 1,
**dadurch gekennzeichnet,**
**dass** die Umschicht- und Auflockerungseinrichtung (6) einen rotierenden, vorzugsweise um eine horizontale und/oder rechtwinklig zur ersten Erstreckungsrichtung (X) verlaufende Achse (Y) rotierenden, Umschichtkörper (7) aufweist.

3. Gärrest-Konditionierer (1) nach Anspruch 1 oder 2,
**dadurch gekennzeichnet,**
**dass** sich die Umschicht- und Auflockerungseinrichtung (6), insbesondere der Umschichtkörper (7), nur über einen Teilabschnitt der Gesamterstreckung der Ablagefläche entlang der ersten Erstreckungsrichtung (X) zwischen Gärrest-Aufgabestelle (4) und Gärrest-Entnahmestelle (5) erstreckt.

4. Gärrest-Konditionierer (1) nach einem der vorigen Ansprüche,
**dadurch gekennzeichnet,**
**dass** die Umschicht- und Auflockerungseinrichtung (6) entlang der ersten Erstreckungsrichtung (X) verfahrbar ist.

5. Gärrest-Konditionierer (1) nach einem der vorigen Ansprüche,
**dadurch gekennzeichnet,**
**dass** der Gärrest-Konditionierer (1) sich entlang der ersten Erstreckungsrichtung (X) erstreckende Führungen (8), insbesondere ein Schienensystem (8), zur Aufnahme der Umschicht- und Auflockerungseinrichtung (6) aufweist.

6. Gärrest-Konditionierer (1) nach einem der vorigen Ansprüche,
**dadurch gekennzeichnet,**
**dass** die Umschicht- und Auflockerungseinrichtung (6) am Gärrest-Konditionierer (1), vorzugsweise an den Führungen (8), durch eine Abstandsänderungseinrichtung (9) zur Veränderung des Abstands der Umschicht- und Auflockerungseinrichtung (6) zur Ablagefläche (2), insbesondere durch ein Schwenkelement (9), aufgenommen ist.

7. Gärrest-Konditionierer (1) nach einem der vorigen Ansprüche,
**dadurch gekennzeichnet,**
**dass** er einen Förderer (10) zur Aufgabe der Gärreste (3) auf die Ablagefläche (2) aufweist.

8. Gärrest-Konditionierer (1) nach einem der vorigen Ansprüche,
**dadurch gekennzeichnet,**
**dass** die Ablagefläche (2) eine Heizeinrichtung (12) und/oder Wärmedämmung aufweist.

9. Gärrestkonditionierer (1) nach einem der vorigen Ansprüche,
**dadurch gekennzeichnet,**
**dass** die Ablagefläche (2) eine Belüftungseinrichtung (11) zur Belüftung der Gärreste (3) aufweist.

10. Verfahren zur Konditionierung von Gärresten (3) oder Klärschlamm (3), wobei Gärreste (3) im Bereich einer Gärrest-Aufgabestelle (4) einem Gärrest-Konditionierer (1) aufgegeben werden und im Bereich einer von der Gärrest-Aufgabestelle (4) entlang einer ersten Erstreckungsrichtung (X) einer Ablagefläche (2) des Gärrest-Konditionierers (1) für die Gärreste (3) beabstandeten Gärrest-Entnahmestelle (5) dem Gärrest-Konditionier (1) entnommen werden, wobei die Gärreste (3) von der Gärrest-Aufgabestelle (4) zur Gärrest-Entnahmestelle, (5) entlang der ersten Erstreckungsrichtung (X) über die Ablagefäche (2) gefördert werden, wobei eine Umschicht- und Auflockerungseinrichtung (6) sowohl die Förderung als auch eine Umschichtung, Auflockerung, einen Aufschluss und/oder eine Homogenisierung der Gärreste (3) bewirkt.

11. Verfahren nach Anspruch 10,
**dadurch gekennzeichnet,**
**dass** die Umschicht- und Auflockerungseinrichtung (6) zeitgleich nur auf einem Teilabschnitt der Gesamterstreckung der Ablagefläche (2) entlang der ersten Erstreckungsrichtung (X) zwischen Gärrest-Aufgabestelle (4) und Gärrest-Entnahmestelle (5) auf die Gärreste (3) einwirkt.

12. Verfahren nach einem der Ansprüche 10 oder 11,
**dadurch gekennzeichnet,**
**dass** die Umschicht- und Auflockerungseinrichtung (6) mit einem rotierenden, vorzugsweise um eine horizontale und/oder rechtwinklig zur ersten Erstreckungsrichtung (X) verlaufende Achse (Y) rotierenden, Umschichtkörper (7) auf die Gärreste (3) einwirkt.

13. Verfahren nach Anspruch 12,
**dadurch gekennzeichnet,**
**dass** die Rotationsrichtung (R) des Umschichtkörpers (7) so orientiert ist, dass der obere Scheitelpunkt des Umschichtkörpers (7) sich in Richtung der Gärrest-Entnahmestelle (5) bewegt.

14. Verfahren nach einem der Ansprüche 10 bis 13,
**dadurch gekennzeichnet,**
**dass** die Gärreste (3) durch die Umschicht- und Auflockerungseinrichtung (6) aus dem Gärrest-Konditionierer (1) ausgetragen werden.

15. Anlage, umfassend eine Mehrzahl Gärrest-Konditionierer nach einem der Ansprüche 1 bis 9,
**dadurch gekennzeichnet,**
**dass** die Mehrzahl Gärrest-Konditionierer (1) parallel oder/und in Reihe geschaltet sind.
